# EUROPEAN PATENT SPECIFICATION

(11) **EP 4 056 717 B1**
(45) Date of publication and mention of the grant of the patent: **01.05.2024**
(21) Application number: 22161705.3
(22) Date of filing: 11.03.2022
(51) Int. Cl.: C12Q 1/689

(54) **COMPOSITION FOR DIAGNOSIS OF Q FEVER AND METHOD FOR DIAGNOSING Q FEVER USING REAL-TIME PCR**
ZUSAMMENSETZUNG ZUR DIAGNOSE VON Q-FIEBER UND VERFAHREN ZUM DIAGNOSTIZIEREN VON Q-FIEBER MITTELS ECHTZEIT-PCR
COMPOSITION POUR LE DIAGNOSTIC DE LA FIÈVRE Q ET PROCÉDÉ DE DIAGNOSTIC DE LA FIÈVRE Q À L'AIDE D'UN PCR EN TEMPS RÉEL

(30) Priority: 12.03.2021 KR 20210032872
(43) Date of publication of application: 14.09.2022
(73) Proprietor: Industry-Academic Cooperation Foundation, Chosun University, Gwangju 61452 (KR)
(72) Inventor: KIM, Dong Min, 61704 Gwangju (KR); LEE, You Mi, 61736 Gwangju (KR); KIM, Choon Mee, 61414 Gwangju (KR)
(74) Representative: Murgitroyd & Company

(56) References cited:
- CN-B- 103 103 249
- SCHNEEBERGER PETER M. ET AL: "Real-Time PCR with Serum Samples Is Indispensable for Early Diagnosis of Acute Q Fever", CLINICAL AND VACCINE IMMUNOLOGY, vol. 17, no. 2, 1 February 2010 (2010-02-01), pages 286-290, XP55943058, ISSN: 1556-6811, DOI: 10.1128/CVI.00454-09
- TURRA M ET AL: "Diagnosis of Acute Q Fever by PCR on Sera during a Recent Outbreak in Rural South Australia", ANNALS OF THE NEW YORK ACADEMY OF SCIENCES, NEW YORK ACADEMY OF SCIENCES, US, vol. 1078, no. 1, 31 October 2006 (2006-10-31), pages 566-569, XP071403433, ISSN: 0077-8923, DOI: 10.1196/ANNALS.1374.112
- HOOVER T A ET AL: "A Coxiella burnetti repeated DNA element resembling a bacterial insertion sequence", JOURNAL OF BACTERIOLOGY, AMERICAN SOCIETY FOR MICROBIOLOGY, US, vol. 174, no. 17, 1 September 1992 (1992-09-01), pages 5540-5548, XP002539104, ISSN: 0021-9193

## Description

### BACKGROUND OF THE INVENTION

### 1. Field of the invention

The present disclosure relates to a composition for diagnosis of Q fever and a method for diagnosing Q fever using real-time PCR.

### 2. Description of the Prior Art

Q fever is a zoonotic disease caused by infection with the bacteria *Coxiella burnetii* (*C. burnetii*) and is known to be transmitted to humans mainly by inhalation of droplets. In Korea, Q fever has been designated as a class 4 legal infectious disease since 2006, and approximately 10 cases have been reported every year thereafter, but a rapid increase has been shown since 2015.

Since acute Q fever shows only non-specific symptoms, such as fever, chills, and headache, clinicians rarely suspect Q fever in the acute phase of the disease. In addition, a diagnosis thereof is carried out by using serological method, and thus acute and convalescent sera of patients are required for an accurate diagnosis.

Indirect immunofluorescence assay (IFA), which is most frequently used for the diagnosis of acute Q fever patients, shows very low sensitivities of 7% and 26% on the diagnosis in the first week and second week of symptom onset respectively. Further, Q fever-specific polymerase chain reaction (PCR) has been reported to show a sensitivity of 26-89% on patients with antibody-negative Q fever.

In serological diagnostic methods for Q fever diagnosis, tests need to be performed 7 to 15 days after the onset of symptoms to identify whether an antibody is present, and the sera in both acute and convalescence phases need to be examined for distinguishment from acute or past infections, and thus a final diagnosis can be made only after a patient recovers from Q fever.

After a 2-3-week incubation period, acute Q fever can lead to hepatitis or pneumonia with non-specific fever, and less than 1% of cases can develop myocarditis, pericarditis, encephalitis, or other complications, but the disease can be effectively treated with doxycycline-based antibiotics through prompt diagnosis. Chronic Q fever is developed in 1-5% of the acute Q fever patients and mainly occurs in immunocompromised persons, pregnant women, and persons with valve diseases, vascular diseases, and artificial hip joints. Chronic Q fever in the form of endocarditis, vasculitis, and osteoarthritis may require an antibiotic treatment for about 2 years and a surgical treatment and may show a mortality rate of up to 50% at follow-up therefore early detection and appropriate treatment are essential. The infection with C. *burnetii* during pregnancy is usually asymptomatic, but it can cause complications in both the mother and the fetus. In many cases, the fetus suffers from spontaneous abortion, stillbirth, premature birth, and intrauterine growth retardation, while the mother suffers from chronic Q fever. Although it is known that risks to the fetus can be reduced in pregnant women who develop acute Q fever and are treated with antibiotics, pregnancy-related issues can occur in up to 81% of untreated pregnant women.

In Korea, indirect immunofluorescence assay is dominantly used for the diagnosis of Q fever and C. *burnetii* culture tests are performed. However, these methods are not generally recommended since C. *burnetii* culturing takes a lot of time and effort and is a hazardous task that requires a biosafety level 3 facility.

Therefore, it is of urgent need to develop a novel diagnostic method that can promptly and conveniently diagnose Q fever with high accuracy.

### [Prior Art Document]

### [Patent Document]

(Patent Document 1) Korean Patent Registration No. 10-2200940 Schneeberger Peter M. Et al. Clinical and Vaccine Immunology, Vol.17, No. 2, Feb 2010, pages 286-290 relates to Real_time PCR with serum samples in the early diagnosis of acute Q fever.

CN 103 103 249 relates to a Q fever *Coxiella burnetii* TaqMan fluorescent quantitative PCR detection method.

Turra M et al. Anals of the New York Academy of Sciences, Vol. 7, No. 1, Oct 2006, pages 566-569 relates to diagnosis of acute Q fever by PCR on sera during an outbreak in rural south Australia.

### SUMMARY OF THE INVENTION

The present inventors designed primers and probes for Q fever diagnosis targeting IS1111 of *Coxiella burnetii* and identified that the use of the primers and probes improves detection sensitivity and specificity compared with existing diagnostic methods to enable an early diagnosis of Q fever very promptly and accurately, and therefore completed the present invention.

Accordingly, an aspect of the present disclosure is to provide a composition for detection of Q fever bacteria, the composition including a polynucleotide set containing a primer of SEQ ID NO: 1, a primer of SEQ ID NO: 2, and a probe of SEQ ID NO: 3, or a polynucleotide set containing a primer of SEQ ID NO: 4, a primer of SEQ ID NO: 2, and a probe of SEQ ID NO: 9.

Another aspect of the present disclosure is to provide a kit for diagnosis of Q fever, the kit including the composition for detection of Q fever bacteria of the present disclosure.

Still another aspect of the present disclosure is to provide an information providing method for diagnosis of Q fever by using the kit for diagnosis of Q fever of the present disclosure.

In accordance with an aspect of the present disclosure, there is provided a composition for detection of Q fever bacteria. The composition includes a polynucleotide set containing a primer of SEQ ID NO: 1, a primer of SEQ ID NO: 2, and a probe of SEQ ID NO: 3, or a polynucleotide set containing a primer of SEQ ID NO: 4, a primer of SEQ ID NO: 2, and a probe of SEQ ID NO: 9.

In accordance with another aspect of the present disclosure, there is provided a kit for diagnosis of Q fever. The kit includes the composition for detection of Q fever bacteria of the present disclosure.

In accordance with still another aspect of the present disclosure, there is provided an information providing method for diagnosis of Q fever. The method includes: (1) isolating a nucleic acid from a sample isolated from a patient suspected of having Q fever; (2) performing a PCR amplification using the isolated nucleic acid as a template by the kit for diagnosis of Q fever of the present disclosure; and (3) checking the presence of the IS1111 gene of *Coxiella burnetii* through step (2).

In an embodiment of the present disclosure, the sample in step (1) may be blood, tissue, cells, serum, plasma, saliva, sputum, or urine.

In an embodiment of the present disclosure, the IS1111 gene of *Coxiella burnetii* may contain the nucleotide sequence of SEQ ID NO: 10.

In an embodiment of the present disclosure, the PCR amplification may be selected from the group consisting of conventional polymerase chain reaction (C-PCR), nested polymerase chain reaction (N-PCR), multiple polymerase chain reaction, real-time polymerase chain reaction, real-time quantitative polymerase chain reaction, and reverse transcription polymerase chain reaction.

The composition for detection of Q fever and the method for diagnosing Q fever by using the composition according to the present disclosure can specifically detect the IS1111 gene of *Coxiella burnetii,* and can promptly and accurately diagnose Q fever through a simple method using real-time PCR due to excellent accuracy and sensitivity compared with existing known detection methods.

### BRIEF DESCRIPTION OF THE DRAWINGS

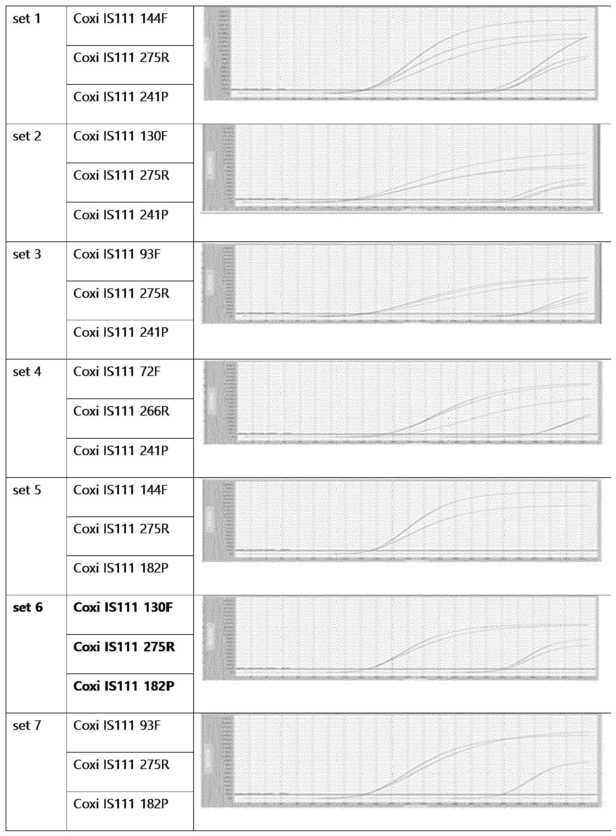

FIG. 1 shows the results of comparative analysis of detection sensitivity through PCR using primers and probes for detection of the IS1111 gene of *Coxiella burnetii,* which were designed according to an embodiment of the present disclosure.

### DETAILED DESCRIPTION OF THE EXEMPLARY EMBODIMENTS

The present disclosure is characterized by providing a novel composition for detection of Q fever bacteria to enable a diagnosis of Q fever promptly and accurately.

While researching a method for accurately and promptly detecting Q fever bacteria for prompt and accurate diagnosis and treatment of the infection with Q fever bacteria causing the onset of Q fever, the present inventors identified that the IS1111 gene of *Coxiella burnetii,* Q fever bacteria, can be used as a new target marker for detection of Q fever bacteria and established primers and probes capable of detecting an infection with Q fever bacteria with high sensitivity and accuracy by specifically binding to the IS1111 gene.

As described with respect to the conventional art as above, indirect immunofluorescence assay is conventionally used for diagnosis of Q fever, but such assay has a problem in the early diagnosis due to very low sensitivities of 7% and 26% on the diagnosis in the first week and second week of symptom onset, respectively, and the serological diagnostic method cannot achieve a substantial early diagnosis since the sera in both acute and convalescence phases need to be examined and thus a final diagnosis can be made only after a patient recovers from Q fever.

In this regard, the present disclosure can provide a composition for detection of Q fever bacteria to enable a prompt and convenient diagnosis of Q fever with high sensitivity and specificity at an early phase, and thus can provide a novel method for diagnosis of Q fever to enable an early diagnosis of Q fever, distinguishment of Q fever from other diseases, a convenient diagnosis through a simple method of PCR, and a diagnosis with excellent accuracy compared with conventional methods.

In an example of the present disclosure, on the basis of various pieces of sequence information on the IS1111 gene of the Q fever bacteria *Coxiella burnetii,* sequence conserved regions in various sequences of the IS1111 gene were analyzed, and thus primers and probes containing various nucleotide sequences capable of detecting the conserved regions were prepared. Out of many primers and probes containing different nucleotide sequences, primers and probes having far superior sensitivity and specificity for the IS1111 gene of *Coxiella burnetii* were established.

Out of the sets for detection of Q fever bacteria, the sets containing the primers and probes established in the present disclosure, one set may include Coxi IS1111 144F primer (5'-GTGGGGTAAAGTGATCTAC-3'; SEQ ID NO: 1), Coxi IS1111 275R primer (5'-CCCATAAACGTCCGATAC-3'; SEQ ID NO: 2), and Coxi IS1111 241P probe (5'-CACACGCTTCCATCACCACG-3'; SEQ ID NO: 3), and another set may include Coxi IS1111 130F primer (5'-TGTGGAATTGATGAGTGG-3'; SEQ ID NO: 4), Coxi IS1111 275R primer (5'-CCCATAAACGTCCGATAC-3'; SEQ ID NO: 2) and Coxi IS1111 182P probe (5'-CTCAGTATGTATCCACCGTAGCCAG-3'; SEQ ID NO: 9).

According to an experiment of the present disclosure, it was identified that many primers and probes designed on the basis of the IS1111 sequences of *Coxiella burnetii* cannot accurately amplify the gene and the use of only the set using primers of SEQ ID NOs: 1 and 2 and a probe of SEQ ID NO: 3 and the set using primers of SEQ ID NOs: 4 and 2 and a probe of SEQ ID NO: 9 can detect the IS1111 gene of *Coxiella burnetii* with specifically high sensitivity.

Therefore, the present disclosure is characterized by providing a composition for detection of Q fever bacteria, the composition including a polynucleotide set containing a primer of SEQ ID NO: 1, a primer of SEQ ID NO: 2, and a probe of SEQ ID NO: 3, or a polynucleotide set containing a primer of SEQ ID NO: 4, a primer of SEQ ID NO: 2, and a probe of SEQ ID NO: 9.

In the present disclosure, the primers and probes can specifically bind to the IS1111 gene of Q fever bacteria, preferably *Coxiella burnetii,* wherein the IS1111 gene of *Coxiella burnetii* contains the nucleotide sequence of SEQ ID NO: 10.

Furthermore, in another example of the present disclosure, a comparative analysis of the detection effect of Q fever bacteria was made through PCR using conventionally known primers for detection of Q fever bacteria and the primers and probe of the present disclosure, and it was identified that PCR using the primers and probe of the present disclosure showed superior sensitivity and specificity compared with PCR using conventional primers.

In an example of the present disclosure, PCR analysis using the detection kit of the present disclosure was performed on samples of patients diagnosed with other diseases than Q fever, and as a result of the analysis, the detection kit of the present disclosure detected reaction products only in the samples of patients diagnosed with Q fever but did not detect any reaction product in the samples of patients with other diseases, indicating that the detection kit of the present disclosure can diagnose Q fever selectively by distinguishing Q fever from other diseases.

Therefore, the composition for detection of Q fever bacteria according to the present disclosure contains the primers and probe of the present disclosure capable of detecting the IS1111 gene of *Coxiella burnetii* with specifically high sensitivity and accuracy, and the composition can not only analyze whether Q fever bacteria are present in a sample to be analyzed but also be used as a diagnostic composition for diagnosis of Q fever.

Hence, the present disclosure can provide a composition for diagnosis of Q fever, the composition including a polynucleotide set containing a primer of SEQ ID NO: 1, a primer of SEQ ID NO: 2, and a probe of SEQ ID NO: 3, or a polynucleotide set containing a primer of SEQ ID NO: 4, a primer of SEQ ID NO: 2, and a probe of SEQ ID NO: 9.

The detection or diagnostic composition of the present disclosure can contain, in addition to the above-described primer pair and probe according to the present disclosure, components usable in the polymerase chain reaction (PCR), for example, a reaction buffer, dNTPs, Mg²⁺ ions, and DNA polymerase.

The reaction buffer may employ 1-10 mM Tris HCl and 10-40 mM KCl (pH 9.0), and dNTPs may employ any one or more selected from dATP, dTTP, dGTP, and dCTP.

The composition may also contain a stabilizer and/or a non-reactive dye for the improvement of experimental convenience, stabilization, and reactivity.

The non-reactive dye needs to be selected from the materials that do not affect the polymerase chain reaction and is intended to be used for analysis or identification using polymerase chain reaction. The materials satisfying those conditions may be used as water-soluble dyes, such as rhodamine, tamra, lax, bromophenol blue, xylene cyanole, bromocresol red, and cresol red.

The composition may be provided in a liquid form and is preferably in a dried state so as to increase stability, storage convenience, and long-term storage properties. The drying may be performed by a known drying method, such as typical room-temperature drying, heating drying, freeze drying, and reduced-pressure drying, and any drying method can be used as long as no ingredient of the composition is lost. The foregoing drying methods may be applied differently depending on the types and amounts of enzymes used.

The composition is prepared in a stabilized state, before use, by mixing in one reaction tube and then carrying out freezing or drying, and thus requires no separate mixing process even during the polymerase chain reaction, thereby preventing errors due to mixing during the reaction and improving stability, reactivity, and storage properties.

The composition may use a commercially available product already containing a reaction buffer, dNTPs, Mg2+ ions, and DNA polymerase, which are components other than the primer pair and probe.

Furthermore, the present disclosure can provide a kit for diagnosis of Q fever, the kit containing the composition of the present disclosure.

The diagnostic kit according to the present disclosure contains a polynucleotide set containing a primer of SEQ ID NO: 1, a primer of SEQ ID NO: 2, and a probe of SEQ ID NO: 3, or a polynucleotide set containing a primer of SEQ ID NO: 4, a primer of SEQ ID NO: 2, and a probe of SEQ ID NO: 9, and thus can promptly diagnose Q fever with high accuracy at an early phase.

For the diagnosis, polymerase chain reaction (PCR) may be used to determine whether Q fever bacteria are contained in a sample to be analyzed, and examples of the polymerase chain reaction include conventional polymerase chain reaction, reverse transcription polymerase chain reaction (RT-PCR), real-time polymerase chain reaction (real-time PCR), and the like. In an example of the present disclosure, real-time polymerase chain reaction was performed.

The diagnostic kit of the present disclosure may further include, in addition to the primer pair and probe of the present disclosure, reverse transcriptase, polymerase, complementary DNA (cDNA) as template DNA for the polymerase chain reaction, and a reaction reagent such as a buffer.

According to an embodiment of the present disclosure, the diagnostic kit of the present disclosure may be used in the typical polymerase chain reaction or real-time polymerase chain reaction, and in such a case, PCR may be performed on the DNA genome of the Q fever bacterium isolated from a sample to be analyzed, or a reverse transcription reaction through an action of reverse transcriptase from the RNA genome of the Q fever bacterium may be separately performed to synthesize complementary DNA (cDNA), which may be then used as template DNA for the polymerase chain reaction.

The products of the polymerase chain reaction may be separated by agarose gel electrophoresis, capillary electrophoresis, or the like, and the diagnosis of Q fever may be determined by checking the presence of DNA having a length corresponding to DNA that is polymerized by the used primer pair and probe.

In the present disclosure, a probe of SEQ ID NO: 3 or SEQ ID NO: 9 capable of specifically binding to the IS1111 gene of the Q fever bacteria *Coxiella burnetii* was used, wherein such a probe may be used by binding of a fluorescent substance and a quencher to both ends thereof. Specifically, a fluorescent substance may be bound to the 5' end of the probe and a quencher may be bound to the 3' end thereof, and a fluorescent substance emitting a particular wavelength, such as red, green, or blue, may be bound to the 5' end of the probe and a black hole quencher (BHQ) may be bound to the 3' end thereof. In a state in which a fluorescent substance and a quencher are bound to the probe, a fluorescence signal is not detected since the quencher absorbs light emitted from the fluorescent substance. On the other hand, when the bound fluorescent substance is separated by exonuclease activity of DNA polymerase during DNA synthesis and elongation, a fluorescence signal can be detected. During the polymerase chain reaction, such a fluorescence signal can be measured in real time, and the presence or absence of the viral gene can be determined on the basis of the increased fluorescence signal. The use of such a probe can be advantageously used in the real-time polymerase chain reaction.

Furthermore, the present disclosure can provide a method for diagnosing an infection with Q fever bacteria and can provide an information providing method for diagnosis of Q fever.

Preferably, the information providing method for diagnosis of Q fever includes: (1) isolating a nucleic acid from a sample isolated from a patient suspected of having Q fever; (2) performing a PCR amplification using the isolated nucleic acid as a template by the diagnostic kit of the present disclosure; and (3) checking the presence of the IS1111 gene of *Coxiella burnetii* through step (2).

Additionally, when RNA is isolated as the nucleic acid from the sample, cDNA is synthesized from the RNA, and real-time PCR amplification using the synthesized cDNA as a template may be performed by using the diagnostic kit of the present disclosure.

The sample may be, but is not limited to, blood, tissue, cells, serum, plasma, saliva, sputum, or urine, which are obtained from mammals.

In the method, the identification of the IS1111 gene of *Coxiella burnetii* may be conducted by at least any one method selected from the group consisting of capillary electrophoresis, DNA chip technology, gel electrophoresis, radioactive measurement, fluorescence measurement, and phosphorescence measurement.

As described above, the use of the polynucleotide set containing a primer of SEQ ID NO: 1, a primer of SEQ ID NO: 2, and a probe of SEQ ID NO: 3 or the polynucleotide set containing a primer of SEQ ID NO: 4, a primer of SEQ ID NO: 2, and a probe of SEQ ID NO: 9, capable of specifically binding to the IS1111 gene of *Coxiella burnetii,* designed in the present disclosure, can promptly detect Q fever bacteria with high sensitivity and accuracy. As a result of analyzing actual patient samples in the examples below, it was identified that a diagnosis can be achieved with a high sensitivity of 80% or more and a high specificity of 100% and a diagnosis can be made with high accuracy for a very short time (within 1 hour).

Hence, the method for diagnosing Q fever and the information providing method for diagnosis of Q fever, each of which uses the composition for detection of Q fever bacteria, designed in the present disclosure, can attain an early diagnosis of Q fever bacteria promptly and accurately, and can monitor the degree of alleviation of symptoms according to the drug administration since the real-time detection of Q fever bacteria is possible, and furthermore, can distinguish Q fever from other diseases with high specificity and thus can also be advantageously used in the selection of accurate therapies.

Hereinafter, the present disclosure will be described in detail through examples. These examples are given for specifically illustrating the present disclosure, and the scope of the present disclosure is not limited thereto.

### <Example 1>

### Target gene selection and primer and probe preparation for Q fever bacteria detection

In order to develop a novel diagnostic method for Q fever diagnosis, the present inventors prepared primers and probes for detection targeting the IS1111 gene of the Q fever bacteria *Coxiella burnetii.*

For target site selection, sequence information on the IS1111 gene of *Coxiella burnetii* was collected, and primers and probes capable of detecting conserved regions were designed through sequencing.

Table 1 below shows various pieces of sequence information for the IS1111 gene of *Coxiella burnetii.*

**TABLE 1**

| Sequence information of IS1111 of *Coxiella burnetii* | | |
|---|---|---|
| GenBank Accession no. | *sp*. | Strain |
| AE016828 | *Coxiella burnetii* | RSA 493 |
| CP000733 | *Coxiella burnetii* | Dugway 5J108-111 |
| CP000890 | *Coxiella burnetii* | RSA 331 |
| CP001019 | *Coxiella burnetii* | CbuG_Q212 |
| CP001020 | *Coxiella burnetii* | CbuK_Q154 |
| CP007555 | *Coxiella burnetii* | Namibia |
| CP013667 | *Coxiella burnetii* | 3262 |
| CP018150 | *Coxiella burnetii* | MSU Goat Q177 |
| HG825990 | *Coxiella burnetii* | Cb175 Guyana |
| KT381466 | *Coxiella burnetii* | EVC13 WGPS 7 |
| KT381467 | *Coxiella burnetii* | EVC13 WGPS 21-22-23 |
| KT391013 | *Coxiella burnetii* | EVC286 |
| KT391014 | *Coxiella burnetii* | EVC282 |
| KT391015 | *Coxiella burnetii* | EVC477 |
| KT391016 | *Coxiella burnetii* | EVC13 |
| KT391017 | *Coxiella burnetii* | EVC477 |
| KT391018 | *Coxiella burnetii* | EVC286 |
| KT391019 | *Coxiella burnetii* | EVC282 |
| KT391020 | *Coxiella burnetii* | EVC13 |
| KT954146 | *Coxiella burnetii* | AuQ31 MLVA |
| LK937696 | *Coxiella burnetii* | Z3055 |
| M80806 | *Coxiella burnetii* | |

As shown in Table 1 above, conserved sequences were searched for the IS1111 sequences of various strains of *Coxiella burnetii,* and various primers and probes in Table 2 below, which were expected to target and amplify the corresponding regions, were designed.

**TABLE 2**

| Primers and probes designed for *Coxiella burnetii* detection | | | | | |
|---|---|---|---|---|---|
| Set | Name | Sequence | Length | SEQ | Reaction |
| No. | | | | ID NO | product (bp) |
| 1 | Coxi IS1111 144F | GTGGGGTAAAGTGATCTAC | 19 | 1 | 132 |
| | Coxi IS1111 275R | CCCATAAACGTCCGATAC | 18 | 2 | |
| | Coxi IS1111 241P | CACACGCTTCCATCACCACG | 18 | 3 | |
| 2 | Coxi IS1111 130F | TGTGGAATTGATGAGTGG | 18 | 4 | 146 |
| | Coxi IS1111 275R | CCCATAAACGTCCGATAC | 18 | 2 | |
| | Coxi IS1111 241P | CACACGCTTCCATCACCACG | 20 | 3 | |
| 3 | Coxi IS1111 93F | ACTGGGTGTTGATATTGC | 18 | 5 | 183 |
| | Coxi IS1111 275R | CCCATAAACGTCCGATAC | 18 | 2 | |
| | Coxi IS1111 241P | CACACGCTTCCATCACCACG | 18 | 3 | |
| 4 | Coxi IS1111 72F | GTCCATGAAAGATATTAAAATACTG | 25 | 6 | 195 |
| | Coxi IS1111 266R | GTCCGATACCAATGGTTC | 18 | 7 | |
| | Coxi IS1111 241P | CACACGCTTCCATCACCACG | 18 | 3 | |
| 5 | Coxi IS1111 144F | GTGGGGTAAAGTGATCTAC | 19 | 8 | 132 |
| | Coxi IS1111 275R | CCCATAAACGTCCGATAC | 18 | 2 | |
| | Coxi IS1111 182P | CTCAGTATGTATCCACCGTAGCCAG | 25 | 9 | |
| 6 | Coxi IS1111 130F | TGTGGAATTGATGAGTGG | 18 | 4 | 146 |
| | Coxi IS1111 275R | CCCATAAACGTCCGATAC | 18 | 2 | |
| | Coxi IS1111 182P | CTCAGTATGTATCCACCGTAGCCAG | 20 | 9 | |
| 7 | Coxi IS1111 93F | ACTGGGTGTTGATATTGC | 18 | 5 | 183 |
| | Coxi IS1111 275R | CCCATAAACGTCCGATAC | 18 | 2 | |
| | Coxi IS1111 182P | CTCAGTATGTATCCACCGTAGCCAG | 20 | 9 | |
| 8 | Coxi IS1111 72F | GTCCATGAAAGATATTAAAATACTG | 25 | 6 | 195 |
| | Coxi IS1111 266R | GTCCGATACCAATGGTTC | 18 | 7 | |
| | Coxi IS1111 182P | CTCAGTATGTATCCACCGTAGCCAG | 20 | 9 | |

### <Example 2>

### Selection of optimal primers and probes for Q fever bacteria detection

The primers and probes for detection of the IS1111 target gene of *Coxiella burnetii* in Table 2, which were designed in Example 1, were tested for sensitivity by performing gradient PCR on the blood of Q fever positive patients.

**TABLE 3**

| Results of detecting IS1111 target gene through gradient PCR using inventive primer and probe sets | | | | | |
|---|---|---|---|---|---|
| Primer and probe set | | Sample Name | 57°C | 59°C | 61°C |
| set **1** | **Coxi IS1111 144F** | **2016-376 buffy** | **32.64** | **34.13** | **34.11** |
| | **Coxi IS1111 275R** | **pc** | **16.61** | **16.80** | **17.02** |
| | **Coxi IS1111 241P** | **nc** | **UD** | **UD** | **UD** |
| set 2 | Coxi IS1111 130F | 2016-376 buffy | 34.53 | 36.81 | 36.09 |
| | Coxi IS1111 275R | PC | 16.09 | 14.84 | 15.17 |
| | Coxi IS1111 241P | nc | UD | UD | UD |
| set 3 | Coxi IS1111 93F | 2016-376 buffy | 36.61 | 36.54 | 37.24 |
| | Coxi IS1111 275R | PC | 16.67 | 16.40 | 16.83 |
| | Coxi IS1111 241P | nc | UD | UD | UD |
| set 4 | Coxi IS1111 72F | 2016-376 buffy | 37.88 | 37.11 | UD |
| | Coxi IS1111 266R | PC | 19.14 | 18.45 | 21.28 |
| | Coxi IS1111 241P | nc | UD | UD | UD |
| set 5 | Coxi IS1111 144F | 2016-376 buffy | UD | UD | |
| | Coxi IS1111 275R | PC | 17.22 | 17.28 | |
| | Coxi IS1111 182P | nc | UD | UD | |
| **set 6** | **Coxi IS1111 130F** | **2016-376 buffy** | **33.9** | **34.5** | |
| | **Coxi IS1111 275R** | **pc** | **17.14** | **16.56** | |
| | **Coxi IS1111 182P** | **nc** | **UD** | **UD** | |
| set 7 | Coxi IS1111 93F | 2016-376buffy | 33.76 | UD | |
| | Coxi IS1111 275R | PC | 16.56 | 16.27 | |
| | Coxi IS1111 182P | nc | UD | UD | |
| set 8 | Coxi IS1111 72F | 2016-376 buffy | 35 | UD | |
| | Coxi IS1111 266R | PC | 17.53 | 18.26 | |
| | Coxi IS1111 182P | nc | UD | UD | |

As shown in results in Table 3 and FIG. 1, out of the primer and probe sets in Table 2 above, the use of Set 1 (the primers of SEQ ID NOs: 1 and 2 and the probe of SEQ ID NO: 3) and Set 6 (the primers of SEQ ID NOs: 4 and 2 and the probe of SEQ ID NO: 9) showed a lower ct value compared with the use of the other primer and probe sets, and thus it can be seen that the primers and probes of Set 1 and Set 6 can promptly detect Q fever bacteria (*Coxiella burnetii*) with high sensitivity. It can also be seen that the use of the primers and probes of Set 1 and Set 6 can save both time and cost compared with existing examination methods for Q fever detection and diagnosis and shows excellent detecting effects.

### <Example 3>

### Analysis of Q fever specificity of inventive primers and probes

### <3-1> Analysis of detection specificity to various bacteria

In order to analyze the specificity to Q fever detection of the inventively designed primers and probe for Q fever detection or diagnosis (Set 1 in Table 1), analysis of whether various bacteria as shown in Table 4 were detected was conducted through PCR.

**TABLE 4**

| Results of performing PCR using inventive primers and probes on various bacteria | | | |
|---|---|---|---|
| | Strain name | Strain details | IS1111 Q-PCR (design) |
| 1 | *Acinetobacter baumannii* (genomic species 2) | KCTC2508 | UD |
| 2 | *Aeromonas hydrophila* subsp. *Hydrophila* | KCTC2358 | UD |
| 3 | *Enterobacter aerogenes* | kctc2190 | UD |
| 4 | *Enterobacter sakazakii* | kctc2949 | UD |
| 5 | *Enterococcus faecalis* | ATCC29212 | UD |
| 6 | *Escherichia coli* | ATCC25922 | UD |
| 7 | *Morganella morganell* subsp. *morganii* | KCCM11497 | UD |
| 8 | *Salmonella enterica* | kctc12456 | UD |
| 9 | *Serratia marcescens* | kctc12457 | UD |
| 10 | *Staphylococcus aureus* subsp. *Aureus* (MRSA) | KCCM40510(ATCC33591 ) | UD |
| 11 | *Streptococcus pyogenes* | KCTC3208 | UD |
| 12 | *Streptococcus agalactiae* | KCCM40417 (ATCC13813 ) | UD |
| 13 | *Vibrio cholerae* | KCCM41626 | UD |
| 14 | *Vibrio parahaemolyticus* | KCCM11965 (ATCC17802 ) | UD |
| 15 | *Vibrio vulnificus* | KCTC 2962 (ATCC27562) | UD |
| 16 | *Staphylococcus aureu* | ATCC 29213 | UD |
| 17 | *Staphylococcus epidermidis* | KCTC1917 (ATCC12228) | UD |
| 18 | *Staphylococcus saprophyticus* subsp. *Saprophyticus* | KCTC3345 (ATCC15305) | UD |
| 19 | *Salmonella typhimurium* | KCTC 1925 | UD |
| 20 | *Klebsiella pneumoniae* | KCTC2242 (ATCC13883) | UD |
| 21 | *Shigella sonnei* | KCTC2518 (ATCC25931) | UD |
| 22 | *Pseudomonas aeruginosa* | ATCC27853 (ATCC27853 ) | UD |
| 23 | *Staplylococcus mitis* | KCTC 3556(ATCC49456) | UD |
| 24 | *Staplylococcus sanguinis* | KCTC3284 (ATCC10556) | UD |
| 25 | *Streptococcus sobrinus* | KCTC3288 (ATCC33478) | UD |
| 26 | *O. tsutsugamushi* Boryong | | UD |
| 27 | *O. tsutsugamushi* Karp | | UD |
| 28 | *O. tsutsugamushi* Kato | | UD |
| 29 | *O. tsutsugamushi* Gilliam | | UD |
| 30 | *Rickettsia honei* | | UD |
| 31 | *Rickettsia prowazekii* | | UD |
| 32 | *Rickettsia rickettsii* | | UD |
| 33 | *Ehrlichia chaffensis* | | UD |
| 34 | *Anaplasma phagocytophilum* | | UD |
| 35 | *Rickettsia akari* | | UD |
| 36 | *Bartonella henselae* | | UD |
| 37 | *Bartonella elizabethae* | | UD |
| 38 | *Bartonella quintana* | | UD |
| 39 | *Anaplasma ovis* | ATCC^{®} VR-1437^{™} | UD |
| 40 | *Anaplasma marginale* Theiler | ATCC^{®} VR-1436^{™} | UD |
| 41 | *Ehrlichia canis* Ebony X+3 T75 | PTA-5812 | UD |

As shown in results in Table 4 above, the kit (Set 1: the primers of SEQ ID NOs: 1 and 2 and the probe of SEQ ID NO: 3) for detection of the IS1111 target gene of *Coxiella burnetii,* designed by the inventive method, produced no PCR product for other bacteria, and thus it can be seen that the kit has high specificity to detect only the IS1111 gene of *Coxiella burnetii.*

### <3-2> Analysis of specificity of inventive kit for Q fever diagnosis on blood samples derived from patients with Q fever and other diseases

The present inventors performed specificity analysis on the inventive kit for Q fever diagnosis, through conventional PCR analysis using the primers of Set 1 to Set 4 disclosed in Table 1, on the blood samples obtained from patients diagnosed with various diseases and Q fever described in Table 5 below.

**TABLE 5**

| NO. | LAB NO. | Disease type | IS1111 C-PCR | | | |
|---|---|---|---|---|---|---|
| | | | Diagnostic kit (Set 1) | d Diagnostic kit (Set 2) | d Diagnostic kit (Set 3) | d Diagnostic kit (Set 4) |
| | | | IS1111 144F | IS1111 130F | IS1111 93F | IS1111 72F |
| | | | IS1111 275R | IS1111 275R | IS1111 275R | IS1111 266R |
| 1 | 2018-449 | viral menigitis | 0 | 0 | 0 | 0 |
| 2 | 2018-1196 | tsutsugamushi | 0 | 0 | 0 | 0 |
| 3 | 2019-039 | OM | 0 | 0 | 0 | 0 |
| 4 | 2019-050 | 1) Allegy 2) rashonleg | 0 | 0 | 0 | 0 |
| 5 | 2019-101 | FUO | 0 | 0 | 0 | 0 |
| 6 | 2019-103 | FUO | 0 | 0 | 0 | 0 |
| 7 | 2019-111 | 1) FUO 2) unknown origin infection 3) r/o TBD 4) DM | 0 | 0 | 0 | 0 |
| 8 | 2019-206 | Q fever | 1 | 0 | 0 | 0 |

As shown in results in Table 5 above, when the inventive Q fever diagnostic kit (Set 1; the primers of SEQ ID NOs: 1 and 2 and the probe of SEQ ID NO: 3) was used, the IS1111 gene of *Coxiella burnetii* was detected only in the sample derived from the patient with *Coxiella burnetii* but was not detected in the samples derived from the patients with the other diseases. In addition, such results were not shown in the groups using the diagnostic kits containing the primers and probes of Set 2, Set 3 and Set 4, but shown only in the diagnostic kit of Set 1 containing the primers of SEQ ID NOs: 1 and 2 and the probe of SEQ ID NO: 3.

Therefore, it can be seen that the inventively designed primers and probe of Set 1 can detect the IS1111 gene of *Coxiella burnetii* with high specificity.

### <Example 4>

### Analysis of PCR detection accuracy using samples isolated from Q fever patients

### <4-1> Sensitivity analysis

Samples were collected and used from patients who were clinically suspected of Q fever and confirmed positive by nested PCR targeting IS1111 or were diagnosed with Q fever due to a 4-fold increase in Q fever antibody titer. The samples were obtained from 15 patients with Q fever in 2016-2019. Respective PCR methods described in Table 6 below were performed by the methods described in Table 7 below.

**TABLE 6**

| Lab no. | Sample | IS1111 N-PCR | phase1 IgG /IgM | phase2 IgG /IgM | 16s N-PCR semi-1 | 16s N-PCR semi-2 | Q-PCR IS1111 _reference | Q-PCR IS1111 inventive diagnostic kit (Set 1) |
|---|---|---|---|---|---|---|---|---|
| 2016-205 | Buffy | Positive | - | - | 0 | 0 | Undetected | Undetected |
| 2016-376 | Buffy | Positive | - | - | Positive | 0 | 38.74 | 35.86 |
| 2017-289 | WB | Positive | - | - | 0 | 0 | 40.71 | 40.33 |
| 2017-289 | Buffy | Positive | - | - | 0 | 0 | Undetected) | Undetected |
| 2017-370 | Plasma | Positive | - | - | Positive | Positive | 36.98 | 33.24 |
| 2017-370 | WB | Positive | - | - | Positive | Positive | 40.49 | 35.01 |
| 2017-370 | Buffy | Positive | - | - | Positive | 0 | 42.15 | 37.30 |
| 2017-512 | Plasma | Positive | - | - | 0 | 0 | 36.80 | 32.21 |
| 2017-512 | WB | Positive | - | - | 0 | 0 | 39.69 | 34.16 |
| 2017-512 | Buffy | Positive | - | - | 0 | 0 | 42.03 | 35.09 |
| 2018-319 | WB | Positive | - | - | Positive | Positive | 31.63 | 27.73 |
| 2018-319 | Serum | Positive | - | - | Positive | Positive | 32.71 | 28.57 |
| 2018-793 | WB | Positive | - | - | 0 | 0 | Undetected | 36.26 |
| 2019-024 | WB | Positive | - | - | 0 | 0 | 41.43 | 39.44 |
| 2019-032 | WB | Positive | - | - | 0 | 0 | 40.66 | 36.24 |
| 2019-AJ03 | WB | Positive | - | - | 0 | 0 | Undetected | 37.63 |
| 2017-289 | Plasma | 0 | Less than 1:16, Less than 1:16 /less than 1:16, 1:256 | Less than 1:16, Less than 1:16 /1:256, 1:2048 | 0 | 0 | Undetected | 35.80 |
| 2017-578 | WB | 0 | Less than 1:16, 1:128 /1:1024, 1:512 | 1:2048, 1:512 /1:2048 or more, less than 1:16 | 0 | ND* | Undetected | 37.95 |
| 2018-148 | WB | 0 | Less than 1:16, less than 1:16 /1:2048, 1:1024 | Less than 1:16, less than 1:16 /1:2048 or more, 1:512 | 0 | ND | Undetected | 37.75 |
| 2018-525 | WB | 0 | Less than 1:16, Less than 1:16 /1:256, less than 1:16 | Less than 1:16, Less than 1:16 /1:1024, less than 1:16 | 0 | ND | 40.80 | 37.86 |
| 2018-716 | WB | 0 | Less than 1:16, Less than 1:16 /1:256, 1:2048 or more | Less than 1:16, 1:128 /1:512, 1:1024 | 0 | ND | 37.86 | 37.68 |
| 2018-753 | WB | 0 | Less than 1:16, Less than 1:16 /1:2048, 1:512 | Less than 1:16, Less than 1:16 /1:2048, 1:16 | 0 | ND | 41.35 | 37.46 |

| | | | | | | | | |
|---|---|---|---|---|---|---|---|---|
| * ND: not done WB: Whole blood | | | | | | | | |

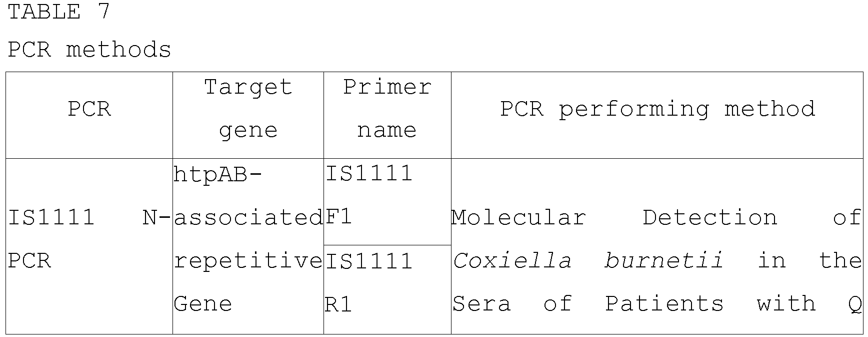

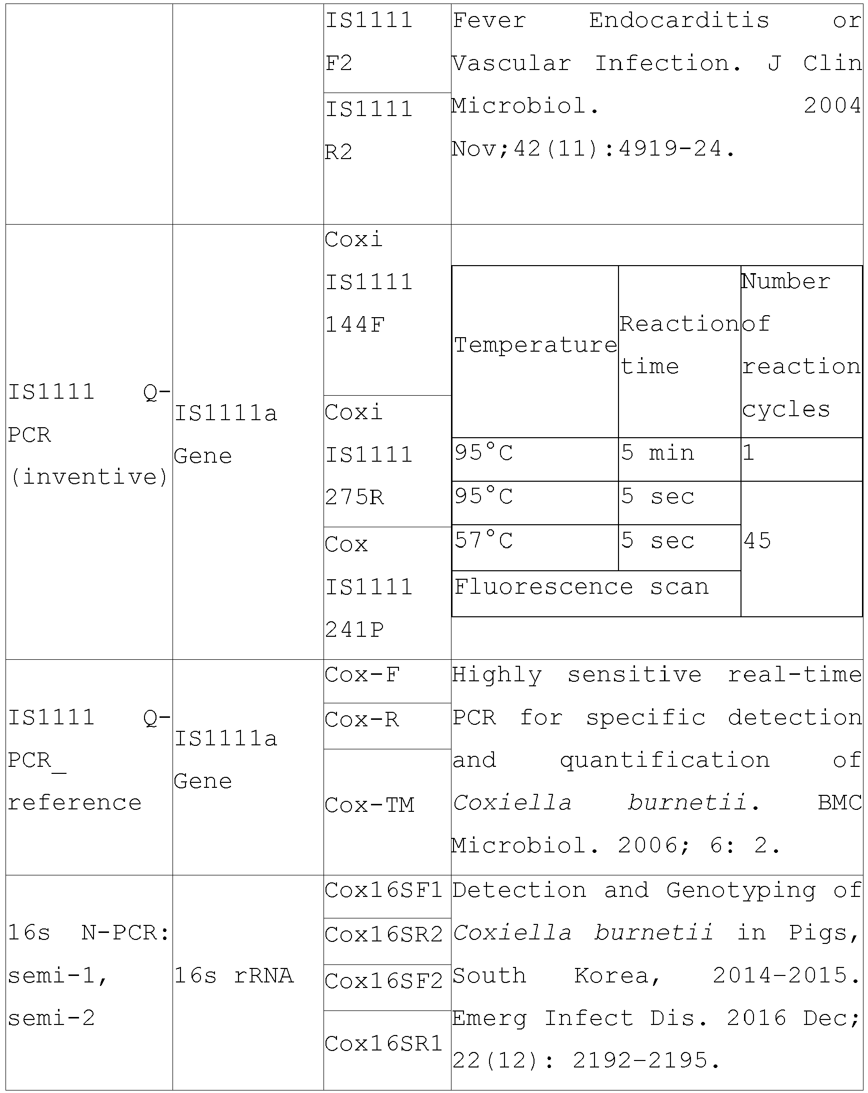

As shown in results in Table 6 above, as result of analyzing the IS1111 target gene of *Coxiella burnetii* by real-time PCR using the inventively designed primers and probe (Set 1) on the samples derived from actual Q fever patients, the target gene IS1111 gene of *Coxiella burnetii* could be detected in each of the samples derived from actual patients, indicating that the real-time PCR method using the inventive detection set showed better detection sensitivity than other existing detection methods. Hence, the real-time PCR method using the inventive kit can detect the IS1111 gene of *Coxiella burnetii* in samples where the gene had not been detected by other existing detection methods. Specifically, the IS1111 N-PCR method (Molecular Detection of Coxiella burnetii in the Sera of Patients with Q Fever Endocarditis or Vascular Infection. J Clin Microbiol. 2004 Nov;42(11):4919-24) showed a sensitivity of 72.7% (16/22). In cases of real-time PCR with a Ct cut-off of 38, the existing Q-PCR IS1111 method (reference Highly sensitive real-time PCR for specific detection and quantification of *Coxiella burnetii.* BMC Microbiol. 2006; 6:2) showed a sensitivity of 22.7% (5/22), whereas the inventive method showed a significantly high sensitivity
of 81.2% (18/22).

It can be therefore seen from these results that the inventively designed primers and probe for Q fever diagnosis to detect the IS1111 gene of *Coxiella burnetii* can diagnose Q fever with excellent sensitivity.

### <4-2> Specificity analysis

Samples for checking specificity were samples collected from 2015 to 2019, and PCR analysis was performed on the samples obtained from patients diagnosed with other diseases. For PCR analysis, a method using the inventively designed primers and probes of Set 1 and an existing PCR method as a control group were used.

**TABLE 8**

| Diagnosis | Lab no. | Spec imen s | IS111 1 N-PCR | Q-PCR IS1111 _Reference | Q-PCR IS1111 _design (inventive) |
|---|---|---|---|---|---|
| OM (osteomyelitis) | 2019-039 | WB | 0 | 32.71 | 43.87 |
| Anaplasmosis | 2018-914 | WB | 0 | 33.12 | Undetermine d |
| FUO (Fever of unknown origin) | 2019-101 | WB | 0 | 36.93 | 40.86 |
| Tsutsugamushi | 2018-1061 | WB | 0 | 37.76 | Undetermine d |
| Tsutsugamushi | 2018-1062 | WB | 0 | 38.01 | Undetermine d |
| Tsutsugamushi | 2018-1077 | WB | 0 | 38.21 | Undetermine d |
| Fever, unspecified | 2019-111 | WB | 0 | 38.45 | Undetermine d |
| Tsutsugamushi | 2018-1030 | WB | 0 | 38.97 | Undetermine d |
| Anaplasmosis | 2018-792 | WB | 0 | 39.08 | Undetermine d |
| Viral menigitis | 2018-449 | WB | 0 | 39.33 | Undetermine d |
| Q fever | 2019-050 | WB | 0 | Undetermine d | 39.61 |
| Tsutsugamushi | 2019-103 | WB | 0 | 39.86 | Undetermine d |
| Tsutsugamushi | 2018-1196 | WB | 0 | 39.90 | Undetermine d |
| Q fever | 2019-110 | WB | 0 | 40.31 | 40.16 |
| Anaplasmosis | 2018-721 | WB | 0 | 41.03 | Undetermine d |
| Fever, unspecified | 2019-095 | WB | 0 | 41.39 | Undetermined |
| Anaplasmosis | 2018-944 | WB | 0 | 43.17 | Undetermine d |
| Tsutsugamushi | 2018-1031 | WB | 0 | Undetermine d | Undetermine d |
| Fever, unspecified | 2019-109 | WB | 0 | Undetermine d | 39.45 |
| Scrub typhus | 2018-1288 | seru m | 0 | Undetermine d | Undetermine d |
| Tsutsugamushi | 2018-1167 | WB | 0 | Undetermine d | Undetermine d |
| Tsutsugamushi | 2018-1176 | WB | 0 | Undetermine d | Undetermine d |
| Tsutsugamushi | 2018-1151 | WB | 0 | Undetermine d | Undetermine d |
| Tsutsugamushi | 2018-1177 | WB | 0 | Undetermine d | Undetermine d |
| Tsutsugamushi | 2018-1227 | WB | 0 | Undetermine d | Undetermine d |
| Tsutsugamushi | 2018-1289 | WB | 0 | Undetermine d | Undetermine d |
| Tsutsugamushi | 2018-1201 | WB | 0 | Undetermine d | Undetermine d |
| Fever, unspecified | 2019-112 | WB | 0 | Undetermine d | Undetermine d |
| acute leukemia | 2018-1282 | WB | 0 | Undetermine d | Undetermine d |
| viral meningitis | 2018-774 | WB | 0 | Undetermine d | Undetermine d |
| bacteremia | 2018-981 | WB | 0 | Undetermined | Undetermine d |
| HAV | 2018-999 | WB | 0 | Undetermine d | Undetermine d |
| acute pyelonephriti s | 2019-029 | WB | 0 | Undetermine d | Undetermine d |
| HAV | 2019-147 | WB | 0 | Undetermine d | Undetermine d |
| Tsutsugamushi | 2018-1078 | WB | 0 | Undetermine d | Undetermine d |
| Tsutsugamushi | 2018-1079 | WB | 0 | Undetermine d | Undetermine d |
| Tsutsugamushi | 2018-1200 | WB | 0 | Undetermine d | Undetermine d |
| Tsutsugamushi | 2018-1202 | WB | 0 | Undetermine d | Undetermine d |
| Anaplasmosis | 2018-383 | WB | 0 | Undetermine d | Undetermine d |
| Anaplasmosis | 2018-527 | WB | 0 | Undetermine d | Undetermine d |
| Anaplasmosis | 2018-541 | WB | 0 | Undetermine d | Undetermine d |
| Anaplasmosis | 2018-601 | WB | 0 | Undetermine d | Undetermine d |
| Rt. hand cellulitis | 2019-098 | WB | 0 | Undetermine d | Undetermine d |

As shown in analysis results in Table 8 above, PCR using a kit having the inventively designed primers and probe (Set 1) showed a specificity of almost 100%, but the group employing the existing method (Q-PCR IS1111_Reference) showed a specificity of about 90%, indicating that the use of the inventive detection kit can detect Q fever with higher specificity than the existing method.

### <4-3> Analysis of accuracy using patient samples

In addition, PCR was performed on each patient sample, and the Q fever accuracy was analyzed through sensitivity and specificity analysis based on the results for each experimental group. As control groups for the group using the inventive detection kit of Set 1, the IS1111 N-PCR group and the Q-PCR IS1111_Reference group used as control groups in the above examples were used. The results are shown in Table 9.

**TABLE 9**

| Results of analyzing detection sensitivity and specificity | | | | | | |
|---|---|---|---|---|---|---|
| | IS1111 N-PCR | | Q-PCR IS1111_Reference | | Q-PCR IS1111_Design | |
| | | | cutoff ≤ 38 | | cutoff ≤ 38 | |
| | Positive control | Negative control | Positive control | Negative control | Positive control | Negative control |
| Positive | 16 | 0 | 5 | 4 | 18 | 0 |
| Negative | 6 | 43 | 17 | 39 | 4 | 43 |
| Sensitivity ( % ) | 72.7 | | 22.7 | | 81.2 | |
| Confidence interval ( % ) | 49.6-88.4 | | 8.7-45.8 | | 59.0-94.0 | |
| Specificity ( % ) | 100 | | 90.7 | | 100 | |
| Confidence interval ( % ) | 89.8-100 | | 76.9-97.0 | | 89.8-100 | |

As shown in analysis results in Table 9 above, when a ct value of 38 or lower was defined for positive, the real-time PCR using the inventively designed primers and probe targeting IS1111 of *Coxiella burnetii* showed a sensitivity of 81.2% and a specificity of 100%, which indicated higher excellent sensitivity and specificity than the existing PCR methods. It can be therefore seen that the use of the inventive primers and probe targeting IS1111 of *Coxiella burnetii* can promptly and conveniently diagnose Q fever with high accuracy.

## Claims

1. A composition for detection of Q fever bacteria, the composition comprising a polynucleotide set containing a primer of SEQ ID NO: 1, a primer of SEQ ID NO: 2, and a probe of SEQ ID NO: 3, or a polynucleotide set containing a primer of SEQ ID NO: 4, a primer of SEQ ID NO: 2, and a probe of SEQ ID NO: 9.

2. A kit for diagnosis of Q fever, the kit comprising the composition of claim 1.

3. An information providing method for diagnosis of Q fever, the method comprising:
(1) isolating a nucleic acid from a sample isolated from a patient suspected of having Q fever;
(2) performing a PCR amplification using the isolated nucleic acid as a template by the kit of claim 2; and
(3) checking the presence of the IS1111 gene of *Coxiella burnetii* through step (2).

4. The method of claim 3, wherein the sample in step (1) is blood, tissue, cells, serum, plasma, saliva, sputum, or urine.

5. The method of claim 3, wherein the IS1111 gene of *Coxiella burnetii* contains the nucleotide sequence of SEQ ID NO: 10.

6. The method of claim 3, wherein the PCR amplification is selected from the group consisting of conventional polymerase chain reaction (C-PCR), nested polymerase chain reaction (N-PCR), multiple polymerase chain reaction, real-time polymerase chain reaction, real-time quantitative polymerase chain reaction, and reverse transcription polymerase chain reaction.

## Patentansprüche

1. Eine Zusammensetzung zum Nachweis von Q-Fieber-Bakterien, wobei die Zusammensetzung einen Polynukleotidsatz, der einen Primer der SEQ ID NO: 1, einen Primer der SEQ ID NO: 2 und eine Sonde der SEQ ID NO: 3 enthält, oder einen Polynukleotidsatz, der einen Primer der SEQ ID NO: 4, einen Primer der SEQ ID NO: 2 und eine Sonde der SEQ ID NO: 9 enthält, beinhaltet.

2. Ein Kit zur Diagnose von Q-Fieber, wobei das Kit die Zusammensetzung gemäß Anspruch 1 beinhaltet.

3. Ein informationslieferndes Verfahren zur Diagnose von Q-Fieber, wobei das Verfahren Folgendes beinhaltet:
(1) Isolieren einer Nukleinsäure aus einer Probe, die aus einem Patienten isoliert wurde, bei dem der Verdacht besteht, dass er Q-Fieber hat;
(2) Durchführen einer PCR-Amplifikation unter Verwendung der isolierten Nukleinsäure als eine Matrize durch das Kit gemäß Anspruch 2; und
(3) Überprüfen des Vorhandenseins des IS1111-Gens von *Coxiella burnetii* durch Schritt (2).

4. Verfahren gemäß Anspruch 3, wobei es sich bei der Probe in Schritt (1) um Blut, Gewebe, Zellen, Serum, Plasma, Speichel, Sputum oder Urin handelt.

5. Verfahren gemäß Anspruch 3, wobei das IS1111-Gen von *Coxiella burnetii* die Nukleotidsequenz SEQ ID NO: 10 enthält.

6. Verfahren gemäß Anspruch 3, wobei die PCR-Amplifikation aus der Gruppe ausgewählt ist, die aus Folgendem besteht: konventioneller Polymerase-Kettenreaktion (C-PCR), verschachtelter Polymerase-Kettenreaktion (N-PCR), multipler Polymerase-Kettenreaktion, Echtzeit-Polymerase-Kettenreaktion, quantitativer Echtzeit-Polymerase-Kettenreaktion und reverser Transkriptase-Polymerase-Kettenreaktion.

## Revendications

1. Une composition pour la détection de bactéries causant la fièvre Q, la composition comprenant un jeu de polynucléotides contenant une amorce de SEQ ID NO : 1, une amorce de SEQ ID NO : 2, et une sonde de SEQ ID NO : 3, ou un jeu de polynucléotides contenant une amorce de SEQ ID NO : 4, une amorce de SEQ ID NO : 2, et une sonde de SEQ ID NO : 9.

2. Un kit pour le diagnostic de la fièvre Q, le kit comprenant la composition de la revendication 1.

3. Un procédé permettant d'obtenir des informations pour le diagnostic de la fièvre Q, le procédé comprenant :
(1) l'isolement d'un acide nucléique issu d'un échantillon isolé à partir d'un patient suspecté de souffrir de la fièvre Q ;
(2) la réalisation d'une amplification par PCR en utilisant l'acide nucléique isolé en tant que matrice au moyen du kit de la revendication 2 ; et
(3) la vérification de la présence du gène IS1111 de *Coxiella burnetii* par le biais de l'étape (2).

4. Le procédé de la revendication 3, où l'échantillon à l'étape (1) est du sang, du tissu, des cellules, du sérum, du plasma, de la salive, des expectorations, ou de l'urine.

5. Le procédé de la revendication 3, où le gène IS1111 de *Coxiella burnetii* contient la séquence nucléotidique de SEQ ID NO : 10.

6. Le procédé de la revendication 3, où l'amplification par PCR est sélectionnée dans le groupe composé de la réaction en chaîne par polymérase classique (C-PCR), la réaction en chaîne par polymérase nichée (N-PCR), la réaction en chaîne par polymérase multiple, la réaction en chaîne par polymérase en temps réel, la réaction en chaîne par polymérase quantitative en temps réel, et la réaction en chaîne par polymérase à transcription inverse.
